# EUROPÄISCHE PATENTANMELDUNG

(11) **EP 1 273 261 A1**
(43) Veröffentlichungstag der Anmeldung: **08.01.2003**
(21) Anmeldenummer: 02405437.1
(22) Anmeldetag: 31.05.2002
(51) Int. Cl.: A61B 5/00

(54) **Gefässprothese mit Messonde**

(30) Priorität: 04.07.2001 EP 01810650
(71) Anmelder: Sulzer Markets and Technology AG, 8401 Winterthur (CH)
(72) Erfinder: Ashton, Tim, West Kilbride, Ayrshire KA23 9HL (GB); Kläui, Erich, 8472 Seuzach (CH); Moser, Urs, 8006 Zürich (CH); Hirt, Felix, 8492 Wila (CH)
(74) Vertreter: Sulzer Management AG

(57) **Zusammenfassung**

Die Kombination einer humanmedizinischen Gefässprothese (1) mit einer an der Gefässprothese (1) verankerbaren Messsonde (2) mit Transponder erlaubt es, die Funktion der Gefässprothese über längere Zeiträume zu überwachen. Mit der Messsonde (2) können geeignete Messgrössen, wie beispielsweise Druckwerte, erfasst und mit Hilfe des Transponders drahtlos an eine Sende-/Empfangsvorrichtung übermittelt werden, wo sie zur Auswertung zur Verfügung stehen.

## Beschreibung

Die Erfindung betrifft eine Gefässprothese mit einer an der Gefässprothese verankerbaren Messsonde mit Transponder, welche Messsonde geeignet ist, Messgrössen zu erfassen, an Hand derer die Funktion der Gefässprothese überprüfbar ist, und welcher Transponder zum Wechselwirken mit einer Sende-/Empfangsvorrichtung ausgebildet ist.

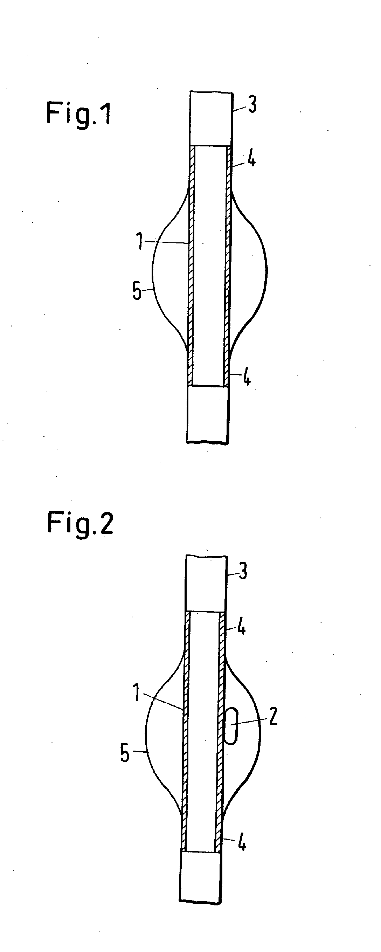

Bei der Behandlung von Gefässverletzungen, -erkrankungen und -anomalien kommen heute in der Humanmedizin Gefässprothesen zum Einsatz. Der Einsatzbereich umfasst dabei nicht nur den intravaskulären Bereich, sondern z.B. auch die Luftröhre, den Verdauungstrakt und die Harnwege. Als typisches Beispiel einer Gefässprothese sei hier das Produkt Anaconda™ der Firma Sulzer Vascutek Ltd. erwähnt, welches bei der Behandlung von Blutgefässerweiterungen (sog. Aneurysmen) eingesetzt wird. Dabei wird die Gefässprothese in das betreffende Gefäss eingeschoben, um die geweitete Stelle zu isolieren und vom Druck zu entlasten, und damit eine weitere Dehnung und den Bruch des betroffenen Gefässes zu verhindern. Fig. 1 zeigt eine solche Gefässprothese im implantierten Zustand. Die Gefässprothese besteht im Wesentlichen aus einem Schlauchstück 1, welches zur Isolation der sackförmigen Gefässerweiterung 5 in das Blutgefäss 3 eingesetzt wurde. Bei intakter Gefässprothese sind die Übergänge 4 vom Schlauchstück 1 auf die Gefässwand beidseits der Gefässerweiterung 5 vollkommen dicht, so dass kein Blut vom Blutgefäss 3 in die Gefässerweiterung 5 gelangen kann. Die Gefässerweiterung 5 ist somit von dem im Gefäss 3 herrschenden Druck entlastet und dehnt sich nicht weiter aus. Im Fall einer Leckage an den Übergängen 4 gelangt jedoch Blut in die Gefässerweiterung 5, worauf sich diese weiter ausdehnt bis sie reisst. Wird dieser Vorgang nicht rechtzeitig entdeckt und unterbrochen, so kann dies für den Patienten tödlich sein. Die ordnungsgemässe Funktion der Gefässprothese, d.h. in diesem Fall die Dichtigkeit der Gefässprothese 1 und der Übergänge 4, ist für den Patienten von entscheidender Bedeutung. Ein geeignetes Überwachungssystem, welches erlaubt, die Dichtigkeit, der implantierten Gefässprothese periodisch oder kontinuierlich zu überwachen, fehlt jedoch im Stand der Technik. Zwar wird im Dokument WO 99/59467 eine Messsonde beschrieben, welche am Ende eines Katheters fixiert ist, und mit welcher Zustandsparameter und Messgrössen, wie beispielsweise Temperatur, Druck usw., lokal im Körperinnern erfasst werden können. Mit der im Dokument WO 99/59467 beschriebenen Messsonde ist es jedoch im Allgemeinen nicht möglich, ohne chirurgischen Eingriff Messungen zur Funktionsüberprüfung von implantierten Gefässprothesen vorzunehmen. Ein weiterer Nachteil der im Dokument WO 99/59467 beschriebenen Messsonde besteht darin, dass die Messsonde für die Messung extra eingeführt und an der Messstelle positioniert werden muss, was einen zusätzlichen Aufwand bedeutet.

Der Erfindung liegt die Aufgabe zu Grunde, unter Vermeidung der aus dem Stand der Technik bekannten Nachteile ein System zur Funktionsüberwachung von Gefässprothesen zur Verfügung zu stellen. Insbesondere soll das System geeignet sein, beginnende Leckagen frühzeitig zu erkennen.

Diese Aufgabe wird durch die in den unabhängigen Ansprüchen definierten Gegenstände der Erfindung gelöst.

Die erfindungsgemässe Gefässprothese umfasst eine an der Gefässprothese verankerbare Messsonde mit Transponder, welche Messsonde geeignet ist, Messgrössen zu erfassen, an Hand derer die Funktion der Gefässprothese überprüfbar ist, und welcher Transponder zum Wechselwirken mit einer Sende-/Empfangsvorrichtung, z.B. einem sog. Interrogator, ausgebildet ist.

Messgrössen, an Hand derer die Funktion der Gefässprothese überprüft werden kann, sind z.B. mechanische Grössen, wie beispielsweise Volumenoder Formänderungen, Kräfte, Drücke u.ä., oder biologische Grössen, wie beispielsweise pH-Werte, Elektrolyt-, Blutgas-, oder Proteinkonzentrationen u.ä.. Die erfindungsgemässe Gefässprothese ermöglicht es, durch periodische, beispielsweise vierteljährliche, Erfassung und Langzeitauswertung der besagten Messgrössen Veränderungen im Zusammenhang mit der implantierten Gefässprothese, wie beispielsweise eine beginnende Leckage, frühzeitig zu erkennen.

Vorzugsweise ist die Messsonde mechanisch mit der Gefässprothese verbunden oder in der Gefässprothese integriert. In einer bevorzugten Ausführungsform ist die Messsonde mittels Befestigungsleinen auf der Oberfläche des Gefässimplantates befestigt.

Vorzugsweise ist die Gefässprothese flexibel und kann zusammen mit der Messsonde via ein Körpergefäss implantiert werden. Dazu wird beispielsweise die zusammengefaltete Gefässprothese und die an derselben verankerte Messsonde in einen Katheter eingesetzt und der derart vorbereitete Katheter in ein Körpergefäss eingeführt und an der für die Implantation vorgesehenen Stelle platziert. Mit Hilfe eines Steuerstabes wird die Gefässprothese und die an derselben verankerte Messsonde aus dem Katheter geschoben, wobei die Gefässprothese bis zur vollen Länge entfaltet wird, und die Messsonde in Ihre Endlage gebracht wird. Das erfindungsgemässe Verfahren hat den Vorteil, dass eine separate Implantation der Messsonde entfällt. Weiter kann die Messsonde mit dem erfindungsgemässen Verfahren in einfacher Weise an Stellen platziert werden, die nach dem Einsetzen der Gefässprothese nur noch operativ zugänglich sind, wie z.B. die äussere Oberfläche der Gefässprothese.

Vorzugsweise ist die Gefässprothese im Wesentlichen schlauchartig ausgebildet und/oder der Form eines Gefässes oder Gefässteiles nachgebildet, um im implantierten Zustand eine krankhafte Gefässerweiterung von innen zu isolieren, wobei die Messsonde auf der äusseren Oberfläche der Gefässprothese vorgesehen ist, so dass die Dichtigkeit der Gefässprothese gegenüber dem erweiterten Gefäss überwachbar ist.

In einer bevorzugten Ausführungsform umfasst die Messsonde eine Hülle und einen Drucksensor, welcher im Inneren der Hülle angeordnet ist, wobei die Hülle mit einem druckübertragenden Medium gefüllt ist. Vorzugsweise ist die Hülle elastisch, so dass bei der Druckmessung die gesamte Oberfläche der Hülle als Druckaufnehmer wirksam ist. Damit wird die für die Druckmessung notwendige Deformation minimiert, was besonders vorteilhaft ist, wenn sich Ablagerungen auf der Messsonde gebildet haben.

Vorzugsweise ist der Transponder als passiver Transponder ausgebildet. Ein passiver Transponder ist eine elektronische Übermittlungsvorrichtung zur drahtlosen Übermittlung von Messwerten ohne eigene Stromversorgung.

Vorzugsweise wird der Drucksensor in die passive Transponderschaltung integriert, beispielsweise indem der Transponder einen kapazitiven Drucksensor und eine Induktivität umfasst, wobei der kapazitive Drucksensor und die Induktivität so miteinander verbunden sind, dass sie einen Schwingkreis bilden. Wahlweise kann der passive Transponder auch einen induktiven Drucksensor und eine Kapazität umfassen, die zusammen einen Schwingkreis bilden. Ein derartiger Transponder kann mit minimalem Aufwand realisiert werden. Vorzugsweise umfasst der passive Transponder zusätzlich ein nichtlineares Bauelement, beispielsweise eine Kapazitätsdiode. Durch das nichtlineare Bauelement werden Oberwellen erzeugt, so dass ein zu übermittelnder Messwert mit einer Frequenz übermittelt wird, welche von der Frequenz der Interrogatorstrahlung, mit welcher der Transponder angeregt wird, verschieden ist. Dadurch kann empfängerseits das Transpondersignal besser von der Interrogatorstrahlung getrennt werden.

Eine Sende-/Empfangsvorrichtung, welche speziell für das Wechselwirken mit dem Transponder der erfindungsgemässen Gefässprothese geeignet ist, ist Gegenstand des Anspruchs 12. Die Sende-/Empfangsvorrichtung umfasst eine Interrogatorantenne, die zum Umschliessen des Körperteiles ausgelegt ist, in welchem die Gefässprothese implantiert ist, wie z.B. im Bauch. Das vollständige Umschliessen des Körperteiles durch die Interrogatorantenne hat den Vorteil, dass eine bessere Kopplung zwischen Interrogatorantenne und Transponder erreicht wird, wenn sich die Gefässprothese und der zugehörige Transponder tief im Körperinnern befinden. Vorzugsweise ist die Interrogatorantenne in einer gurtähnlichen Vorrichtung angeordnet. Vorzugsweise umfasst die Interrogatorantenne mindestens eine Primär- und eine Sekundärantenne, wobei die beiden Antennen derart gekoppelt sind, dass das Fernfeld der gekoppelten Antennen abgeschwächt wird. Diese Antennenanordnung hat den Vorteil, dass eine Störung umliegender Geräte und Funkdienste durch die relativ starke Interrogatorstrahlung vermieden werden kann, ohne dass die drahtlose Energieversorgung des Transponders beeinträchtigt wird.

Weitere vorteilhafte Ausführungsformen gehen aus den abhängigen Ansprüchen und der Zeichnung hervor.

Im Folgenden wird die Erfindung an Hand der Ausführungsbeispiele und an Hand der Zeichnung näher erläutert. Es zeigen:
- Fig.1:: einen Längsschnitt durch eine herkömmliche Gefässprothese im implantierten Zustand,
- Fig. 2:: einen Längsschnitt durch eine Gefässprothese gemäss einem ersten Ausführungsbeispiel der vorliegenden Erfindung im implantierten Zustand,
- Fig. 3: einen Schnitt durch eine Messsonde gemäss dem ersten Ausführungsbeispiel der vorliegenden Erfindung,
- Fig. 4: ein Blockschaltbild eines passiven Transponders gemäss dem ersten Ausführungsbeispiel der vorliegenden Erfindung,
- Fig. 5a: einen Schaltplan einer Ausführungsvariante eines passiven Transponders mit integriertem kapazitivem Drucksensor,
- Fig. 5b: einen Schaltplan einer Ausführungsvariante eines passiven Transponders mit integriertem induktivem Drucksensor,
- Fig. 6a: eine Interrogatorantenne gemäss dem ersten Ausführungsbeispiel der vorliegenden Erfindung,
- Fig. 6b: eine Interrogatorantenne mit einer Primär- und zwei Sekundärantennen,
- Fig. 6c: den konstruktiven Aufbau der Interrogatorantenne von Fig. 6b,
- Fig. 7: ein Blockschaltbild eines Interrogators gemäss dem ersten Ausführungsbeispiel der vorliegenden Erfindung,
- Fig. 8a: eine Gefässprothese gemäss einem zweiten Ausführungsbeispiel der vorliegenden Erfindung während der Einführungsphase,
- Fig. 8b: die Gefässprothese gemäss dem zweiten Ausführungsbeispiel in vollständig entfaltetem Zustand,
- Fig. 9a: einen Längsschnitt durch die Gefässprothese gemäss dem zweiten Ausführungsbeispiel zusammengefaltet in einem Katheter,
- Fig. 9b: einen Längsschnitt durch die Gefässprothese gemäss dem zweiten Ausführungsbeispiel während der Entfaltung,
- Fig. 9c: einen Längsschnitt durch die Gefässprothese gemäss dem zweiten Ausführungsbeispiel vollständig entfaltet in einem Aneurysma.

Eine Gefässprothese gemäss einem ersten Ausführungsbeispiel der vorliegenden Erfindung ist in Fig. 2 dargestellt. Die Gefässprothese umfasst einem Schlauchstück 1, welches zur Isolation der sackförmigen Gefässerweiterung 5 in das Blutgefäss 3 eingesetzt wurde, und eine am Schlauchstück 1 verankerte Messsonde 2. Bei intakter Gefässprothese sind die Übergänge 4 vom Schlauchstück 1 auf die Gefässwand beidseits der Gefässerweiterung 5 vollkommen dicht, so dass kein Blut vom Blutgefäss 3 in die Gefässerweiterung 5 gelangen kann. Die Gefässerweiterung 5 ist somit von dem im Gefäss 3 herrschenden Druck entlastet und dehnt sich nicht weiter aus. Die Messsonde 2 ist auf der äusseren Oberfläche des Schlauchstücks 1 verankert, um die Dichtigkeit des Schlauchstücks 1 gegenüber der Gefässerweiterung 5 zu überwachen.

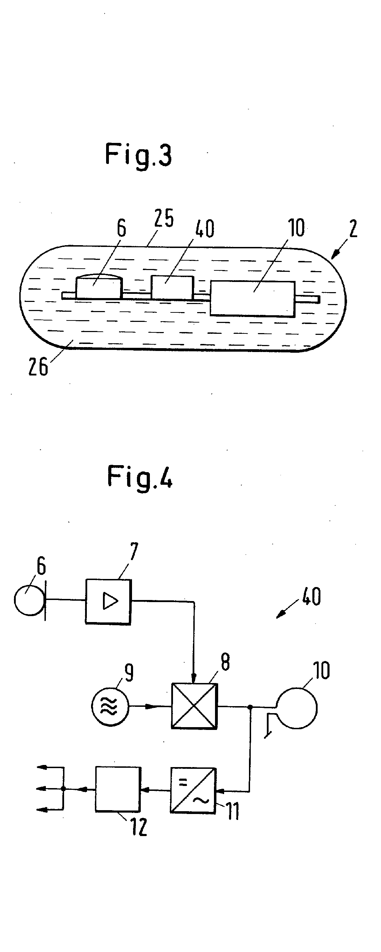

Fig. 3 zeigt eine Messsonde 2 gemäss dem ersten Ausführungsbeispiel der vorliegenden Erfindung. Die Messsonde 2 umfasst eine elastische Hülle 25, einen Drucksensor 6 zur Messung des Druckes im eingeschlossenen Volumen zwischen dem Schlauchstück 1 und der Gefässerweiterung 5 und einen passiven Transponder 40 mit einer Antenne 10 zur Übermittlung der gemessenen Druckwerte. Der Drucksensor 6 ist im Innern der Hülle 25 angeordnet, und die Hülle 25 mit einem druckübertragenden Medium 26, beispielsweise einem Öl oder Gel, gefüllt, welches den auf die Hülle 25 wirkenden Druck auf den Drucksensor 6 überträgt. Diese Anordnung ist besonders vorteilhaft, wenn sich Ablagerungen auf der Messsonde gebildet haben, da die für die Druckmessung notwendigen Deformationen der Hülle minimal sind. Der Drucksensor 6 kann jedoch auch extern angeordnet und mit einem Kabel mit dem Transponder 40 verbunden werden oder in die Messsondenhülle 25 integriert sein. Das Messprinzip des Drucksensors kann piezoresistiver, kapazitiver, induktiver, magnetoelastischer, etc. Art sein.

Der Drucksensor ist im Ausführungsbeispiel mit einem passiven Transponder verbunden. Ein passiver Transponder ist eine elektronische Übermittlungsvorrichtung zur drahtlosen Übermittlung von Messwerten ohne eigene Stromversorgung. Zu seiner Aktivierung wird der Transponder von einer Sende-/Empfangsvorrichtung, im Folgenden auch Interrogator genannt, mit hochfrequenter Strahlung angestrahlt, worauf der Transponder seinerseits ein hochfrequentes Trägersignal aussendet, welches mit der zu übertragenden Information moduliert ist. Dieses Transpondersignal kann im Empfänger der Sende-/Empfangsvorrichtung empfangen und zwecks Gewinnung der übertragenen Information demoduliert werden. Zweckmässigerweise erfolgt die Energieversorgung der Messschaltung ebenfalls aus der vom Transponder aufgenommenen Strahlungsenergie.

Fig. 4 zeigt ein Blockschaltbild eines passiven Transponders 40 gemäss dem ersten Ausführungsbeispiel der vorliegenden Erfindung. Der Transponder 40 wird durch das von einem Interrogator ausgestrahlte hochfrequente Strahlungsfeld mit Energie versorgt. Die Strahlung induziert in einer Antenne 10 eine hochfrequente Spannung, welche in einem Gleichrichter 11 gleichgerichtet und einem Speisemodul 12 zugeführt wird. Im Speisemodul 12 befinden sich ein Speicherkondensator, welcher mit dem vom Gleichrichter gelieferten Gleichstrom aufgeladen wird, sowie Schalter, die den Transponder 40 einschalten, wenn die Spannung am Kondensator die notwendige Betriebsspannung erreicht hat, und den Transponder 40 wieder ausschalten, wenn die minimale Betriebsspannung unterschritten wird. Bei dieser Art der Energieversorgung handelt es sich um eine sequentielle Betriebsweise. Die sequentielle Betriebsweise hat den Vorteil, dass ein wesentlich schwächeres Strahlungsfeld benötigt wird, als beim Duplexbetrieb, wo Interrogator und Transponder gleichzeitig aktiv sind, da die Übertragungsphase in der Regel nur Bruchteile von Sekunden dauert, während für die Ladephase im sequentiellen Betrieb mehrere Sekunden zur Verfügung stehen. Der vom Drucksensor 6 zugeführte Messwert wird in einem Signalaufbereitungsmodul 7 aufbereitet, wo er beispielsweise in einen frequenzmodulierten Hilfsträger umgesetzt oder digitalisiert werden kann. Das derart aufbereitete Signal wird in einen Modulator 8 eingespiesen, wo es den in einem Oszillator 9 erzeugten Träger moduliert. Dieser modulierte Träger wird in die Antenne 10 eingespiesen und von dort als Transpondersignal abgestrahlt. Das Trägersignal kann statt mit dem Oszillator 9 auch durch Rückstrahlung (Reflexion) oder Frequenzvervielfachung oder -teilung aus der Interrogatorstrahlung gewonnen werden. An Stelle eines konventionellen Modulators kann auch eine Modulation der Antennenimpedanz des Transponders angewendet werden. Wird das Trägersignal durch Rückstrahlung aus der Interrogatorstrahlung gewonnen, ergibt dies die sogenannte Last-, Absorptions- oder Rückstreumodulation.

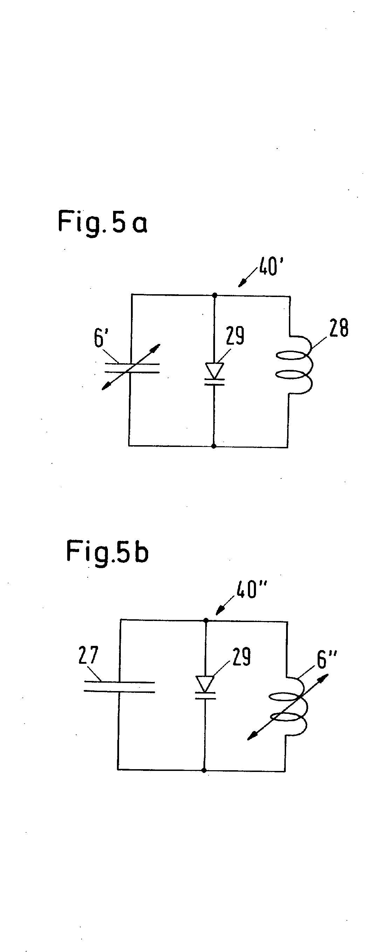

In einer Ausführungsvariante, welche in Fig. 5a gezeigt ist, ist der Drucksensor in die passive Transponderschaltung integriert, indem der Transponder 40' einen kapazitiven Drucksensor 6' und eine Induktivität 28 umfasst. Dabei sind der kapazitive Drucksensor 6' und die Induktivität 28 so miteinander verbunden, dass sie einen Schwingkreis bilden. Wird dieser Schwingkreis durch die hochfrequente Strahlung des Interrogators angeregt, so gibt der Schwingkreis gleichzeitig hochfrequente Strahlung ab, wobei sich die Phase der abgegebenen Strahlung in Abhängigkeit vom Druck ändert. Wahlweise kann der Transponder 40", wie in Fig. 5b gezeigt, auch einen induktiven Drucksensor 6" und eine Kapazität 27 umfassen, wobei der induktive Drucksensor 6" und die Kapazität 27 so miteinander verbunden sind, dass sie einen Schwingkreis bilden. Ein derartiger Transponder 40', 40" kann mit minimalem Aufwand realisiert werden. Vorzugsweise umfasst der passive Transponder 40', 40" zusätzlich ein nichtlineares Bauelement 29, beispielsweise eine Kapazitätsdiode. Durch das nichtlineare Bauelement 29 werden Oberwellen erzeugt, so dass ein zu übermittelnder Messwert mit einer Frequenz übermittelt wird, welche von der Frequenz der Interrogatorstrahlung, mit welcher der Transponder 40', 40" angeregt wird, verschieden ist. Dadurch kann empfängerseits das Transpondersignal besser von der Interrogatorstrahlung getrennt werden.

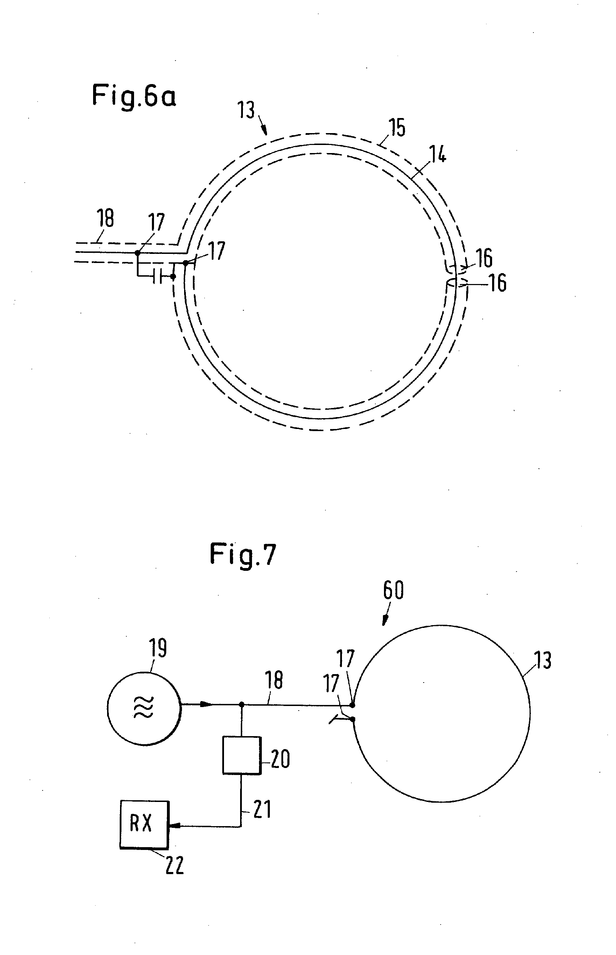

Die Fig. 6a und 7 zeigen eine Interrogatorantenne 13 und ein Blockschaltbild des Interrogators 60 gemäss dem ersten Ausführungsbeispiel der vorliegenden Erfindung. Die Interrogatorantenne 13 ist als elektrisch abgeschirmte Schleifenantenne (loop antenna) aufgebaut. Dank dem Schleifenaufbau ist es möglich, den Körperteil, in welchem die Gefässprothese eingesetzt ist, mit der Antenne zu umschliessen, was den Vorteil hat, dass eine bessere Kopplung zwischen Interrogatorantenne und Transponder erreicht wird, wenn sich die Gefässprothese und der zugehörige Transponder tief im Körperinnern befinden. Die Schleifenantenne 13 ist wie folgt aufgebaut: Der eigentliche Antennendraht 14 befindet sich koaxial im Innern einer schlauchförmigen Abschirmung 15, welche an einer Stelle 16 unterbrochen ist. Durch die unterbrochene Abschirmung wird bewirkt, dass im Nahfeld der Antenne nur das magnetische und nicht das störende elektrische Feld auftritt. Am Einspeisepunkt 17 der Schleifenantenne 13 befindet sich ein Kondensator, mit dem die Antenne auf Resonanz abgestimmt werden kann. Der Einspeisepunkt 17 ist über ein abgeschirmtes Kabel 18 mit einem Hochfrequenzgenerator 19 verbunden. Das vom Transponder abgestrahlte Transpondersignal wird von derselben Antenne 13 empfangen, am Einspeisepunkt 17 abgenommen und über eine Frequenzweiche 20 und ein Kabel 21 zu einem Empfänger 22 geleitet. Die Frequenzweiche 20 verhindert, dass das relativ starke Interrogatorsignal an den Empfängereingang gelangt und diesen beschädigt.

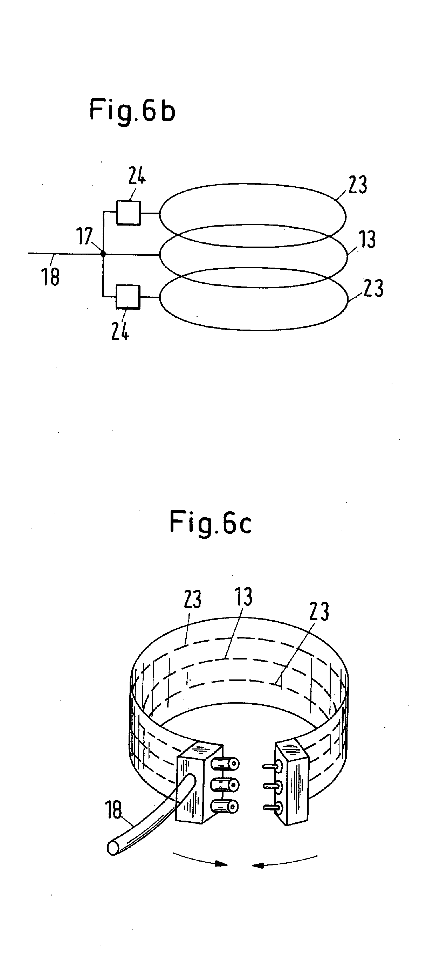

Fig. 6b zeigt eine bevorzugte Ausführungsvariante einer Interrogatorantenne gemäss der vorliegenden Erfindung. Parallel zur Schleifenantenne 13 sind symmetrisch zur Antenne 13 auf beiden Seiten, in einem Abstand, der etwa einem halben bis ganzen Radius der Schleifenantenne 13 entspricht, Kompensationsschleifen 23 angebracht. Die Kompensationsschleifen 23 werden durch Kompensationsströme gespiesen, deren Amplitude insgesamt etwa dem Antennenstrom in der Schleifenantenne 13 entsprechen, und deren Phase gegenüber dem Antennenstrom um 180° verschoben ist. Dadurch wird das Fernfeld der Schleifenantenne 13 weitgehend unterdrückt, ohne dass das für die drahtlose Energieversorgung wichtige Nahfeld wesentlich geschwächt wird. Die Kompensationsströme können beispielsweise vom Einspeisepunkt 17 der Antenne 13 abgenommen und über eine Anpassungsschaltungen 24 den Kompensationsschleifen 23 zugeführt werden. Mit Hilfe der Anpassungsschaltung 24 können die Amplitude und Phase der Kompensationsströme für optimale Unterdrückung des Fernfeldes eingestellt werden. Diese Ausführungsvariante hat den Vorteil, dass Störungen von andern Geräten und Funkdiensten durch das Fernfeld der relativ starken Interrogatorstrahlung, welche für die Energieversorgung des passiven Transponders benötigt wird, weitgehend vermieden werden. In einer weiteren Ausführungsvariante umfasst das Kompensationssystem lediglich eine Kompensationsschleife 23. In einer weiteren Ausführungsvariante sind die Kompensationsschleifen 23 nicht gespiesen.

Vorzugsweise ist die Interrogatorantenne, welche entweder die Antenne 13 allein, oder die Antenne 13 und das in der obigen Ausführungsvariante beschriebenen Kompensationssystem 17, 23 und 24 umfasst, als flexibler Gurt aufgebaut. Der Gurt kann für die Messung um den entsprechenden Körperteil gelegt werden, in welchen die Gefässprothese mit der zugehörigen Messsonde eingesetzt wurde. Eine Interrogatorantenne in der Form eines Gurtes ist in Fig. 6c dargestellt.

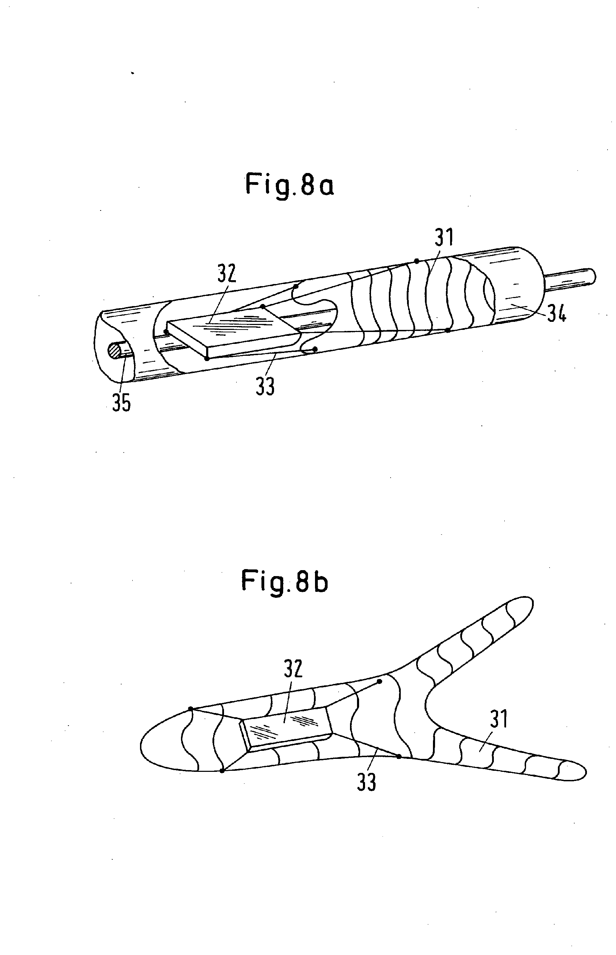

Fig. 8a zeigt eine Gefässprothese gemäss einem zweiten
Ausführungsbeispiel der vorliegenden Erfindung während der Einführungsphase und Fig. 8b die selbe Gefässprothese in vollständig entfaltetem Zustand. In Fig. 8a ist die dicht zusammengefaltete Gefässprothese 31 in einen Katheter 34 eingesetzt. Unmittelbar vor oder nach der zusammengefalteten Gefässprothese 31 befindet sich eine Messsonde 32, welche an beiden Enden mit Hilfe von je zwei Befestigungsleinen 33 auf der äusseren Oberfläche der Gefässprothese 31 befestigt ist. Die Messsonde 32 hat ein schlankes Volumen und ist mit einer biokompatiblen Oberfläche ausgestattet. Die Längen der Messsonde 32 und der hinteren Befestigungsleinen 33 sind etwa gleich gross. Längs des Katheters 34 verläuft ein Steuerstab 35, der dazu dient, die Gefässprothese 31 auszustossen und zu entfalten.

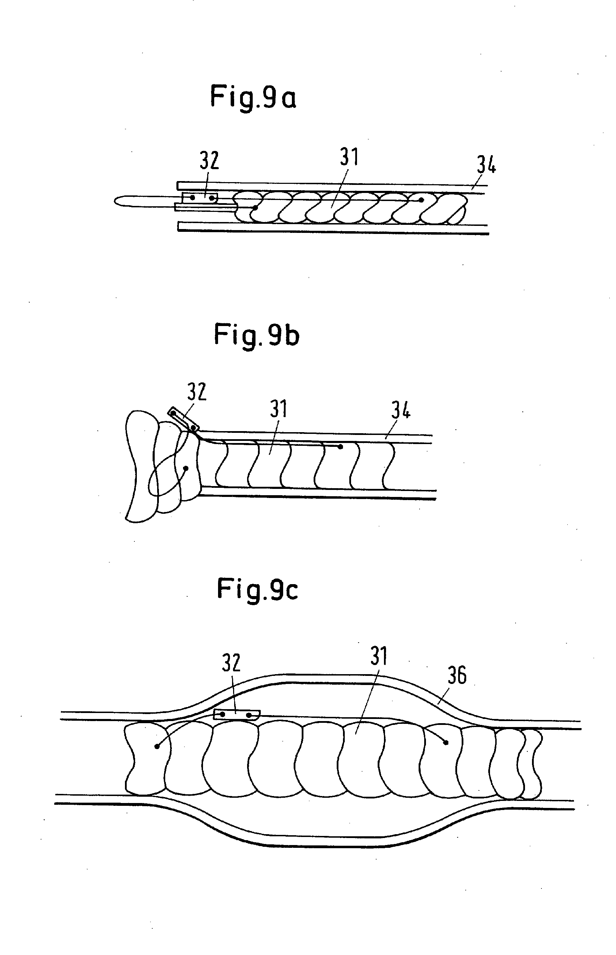

Die Fig. 9a, b und c zeigen das Einsetzen der Gefässprothese gemäss dem zweiten Ausführungsbeispiel der vorliegenden Erfindung in eine Gefässerweiterung, ein sog. Aneurysma. Die dicht zusammengefaltete Gefässprothese 31 wird zusammen mit der Messsonde 32, welche mittels der Befestigungsleinen 33 an der Gefässprothese 31 befestigt ist, in einen Katheter 34 eingesetzt. Fig. 9a zeigt die Gefässprothese 31 zusammengefaltet im Katheter 34. Der derart vorbereitete Katheter 34 wird in ein Körpergefäss eingeführt und an der für die Implantation vorgesehenen Stelle, beispielsweise einem Aneurysma 36, platziert. Mit Hilfe eines Steuerstabes wird die Gefässprothese 31 und die an derselben befestigte Messsonde 32 aus dem Katheter 34 geschoben und die Gefässprothese 31 entfaltet. Fig. 9b zeigt die Gefässprothese 31 während der Entfaltung. Während des Entfaltens der Gefässprothese 31 wird die Messsonde 32 durch die Befestigungsleinen auf die äussere Oberfläche der Gefässprothese 31 gezogen. Nach Abschluss des Entfaltungsvorganges ist die Gefässprothese bis zur vollen Länge entfaltet und die vier Befestigungsleinen 33 sind gestreckt, so dass die Messsonde 32 auf der äusseren Oberfläche der Gefässprothese 31 fixiert ist. Fig. 9c zeigt die Gefässprothese 31 vollständig entfaltet im Aneurysma 36. Das erfindungsgemässe Verfahren hat den Vorteil, dass eine separate Implantation der Messsonde entfällt. Weiter kann die Messsonde mit dem erfindungsgemässen Verfahren in einfacher Weise an Stellen platziert werden, die nach dem Einsetzen der Gefässprothese nur noch operativ zugänglich sind, wie z.B. die äussere Oberfläche der Gefässprothese.

## Patentansprüche

1. Gefässprothese (1, 31) mit einer an der Gefässprothese verankerbaren Messsonde (2, 32) mit Transponder (40, 40', 40"), welche Messsonde (2, 32) geeignet ist, Messgrössen zu erfassen, an Hand derer die Funktion der Gefässprothese (1, 31) überprüfbar ist, und welcher Transponder (40, 40', 40") zum Wechselwirken mit einer Sende-/Empfangsvorrichtung (60) ausgebildet ist.

2. Gefässprothese gemäss Anspruch 1, wobei die Messsonde (2, 32) mechanisch mit der Gefässprothese (1, 31) verbunden ist und/oder in die Gefässprothese (1, 31) integriert ist.

3. Gefässprothese gemäss Anspruch 1 oder 2, wobei die Messsonde (2, 32) mittels mindestens einer Befestigungsleine (33) an der äusseren Oberfläche der Gefässprothese (1, 31) befestigt ist.

4. Gefässprothese gemäss einem der Ansprüche 1 bis 3, wobei die an der Gefässprothese (1, 31) verankerbare Messsonde (2, 32) einen Drucksensor (6, 6', 6") umfasst.

5. Gefässprothese gemäss einem der Ansprüche 1 bis 4, wobei die Wandung der Gefässprothese flexibel ist, und wobei die Gefässprothese und die Messsonde via ein Körpergefäss implantierbar sind.

6. Gefässprothese gemäss einem der Ansprüche 1 bis 5, wobei die Gefässprothese (1, 31) im Wesentlichen schlauchartig ausgebildet ist, um im implantierten Zustand eine krankhafte Gefässerweiterung von innen zu isolieren, und wobei die Messsonde (2, 32) auf der äusseren Oberfläche der Gefässprothese (1, 31) vorgesehen ist, so dass die Dichtigkeit der Gefässprothese (1, 31) gegenüber dem erweiterten Gefäss überwachbar ist.

7. Gefässprothese gemäss einem der Ansprüche 1 bis 6, **gekennzeichnet dadurch, dass** die Messsonde (2, 32) eine Hülle (25) und einen Drucksensor (6, 6', 6") umfasst, welcher im Innern der Hülle (25) angeordnet ist, und dass die Hülle (25) mit einem druckübertragenden Medium (26) gefüllt ist.

8. Gefässprothese gemäss einem der Ansprüche 1 bis 7, wobei der Transponder (40, 40', 40") als passiver Transponder ausgebildet ist.

9. Gefässprothese gemäss Anspruch 8, wobei der passive Transponder (40') einen kapazitiven Drucksensor (6') und eine Induktivität (28) umfasst, und wobei der kapazitive Drucksensor (6') und die Induktivität (28) so miteinander verbunden sind, dass sie einen Schwingkreis bilden.

10. Gefässprothese gemäss Anspruch 8, wobei der passive Transponder (40") einen induktiven Drucksensor (6") und eine Kapazität (27) umfasst, und wobei der induktive Drucksensor (6") und die Kapazität (27) so miteinander verbunden sind, dass sie einen Schwingkreis bilden.

11. Gefässprothese gemäss einem der Ansprüche 8 bis 10, wobei der passive Transponder (40, 40', 40") zusätzlich ein nichtlineares Bauelement (29) umfasst, so dass ein von der Messsonde (2, 32) ermittelter Messwert mit einer Frequenz übermittelt wird, welche von einer Anregungsfrequenz, mit welcher der Transponder (40, 40', 40") angeregt wird, verschieden ist.

12. Sende-/Empfangsvorrichtung (60) zum Wechselwirken mit einem Transponder (40) einer Messsonde (2), welche an einer Gefässprothese (1, 31) gemäss einem der Ansprüche 1 bis 11 vorgesehen ist, welche Sende-/Empfangsvorrichtung (60) eine Interrogatorantenne 13 umfasst, **dadurch gekennzeichnet, dass** die Interrogatorantenne 13 zum Umschliessen desjenigen Körperteiles ausgebildet ist, in welchem die Gefässprothese (1, 31) eingesetzt ist.

13. Sende-/Empfangsvorrichtung gemäss Anspruch 12, wobei die Interrogatorantenne 13 in einer Vorrichtung angeordnet ist, welche die Form eines Gurtes hat.

14. Sende-/Empfangsvorrichtung gemäss Anspruch 12 oder 13, wobei die Interrogatorantenne mindestens eine Primär- und mindestens eine Sekundärantenne umfasst, und wobei die beiden Antennen derart gekoppelt sind, dass das Fernfeld der gekoppelten Antennen abgeschwächt wird.

15. Verfahren zum Implantieren einer Gefässprothese (1, 31) mit einer an der Gefässprothese (1, 31) verankerbaren Messsonde (2, 32) gemäss einem der Ansprüche 1 bis 11, umfassend:
Einsetzen der zusammengefalteten Gefässprothese (1, 31) und der an derselben verankerten Messsonde (2, 32) in einen Katheter (34);
Einführen des Katheters (34) in ein Körpergefäss,
Platzieren des Katheters (34) an der für die Implantation vorgesehenen Stelle,
Herausschieben der Gefässprothese (1, 31) und der an derselben verankerten Messsonde (2, 32) mit Hilfe eines Steuerstabes (35), wobei die Gefässprothese (1, 31) bis zur vollen Länge entfaltet wird, und die Messsonde (2, 32) in ihre Endlage gebracht wird.

16. Verfahren gemäss Anspruch 15, wobei
die Messsonde (2, 32) mittels je zwei Befestigungsleinen (33) an der äusseren Oberfläche des Gefässimplantates (1, 31) befestigt wird,
die Messsonde (2, 32) während des Entfaltens der Gefässprothese (1, 31) durch die Befestigungsleinen (33) auf die äussere Oberfläche der Gefässprothese (1, 31) gezogen wird, und
die vier Befestigungsleinen (33) im Endzustand des Entfaltungsvorganges gestreckt sind, so dass die Messsonde (2, 32) auf der äusseren Oberfläche der Gefässprothese (1, 31) fixiert ist.
